# EUROPEAN PATENT APPLICATION

(11) **EP 4 520 373 A1**
(43) Date of publication of application: **12.03.2025**
(21) Application number: 23873232.5
(22) Date of filing: 27.09.2023
(51) Int. Cl.: A61M 15/00, A61M 11/00, A24F 40/05, A24F 40/10, H03H 9/02

(54) **AEROSOL GENERATION DEVICE**

(30) Priority: 28.09.2022 KR 20220123021
(71) Applicant: KT&G Corporation, Daedeok-gu Daejeon 34337 (KR); Industry Foundation of Chonnam National University, Gwangju 61186 (KR)
(72) Inventor: JUNG, Yongmi, Daejeon 34128 (KR); KIM, Woo Hyuk, Gwangju 62324 (KR); PARK, Jinsoo, Gwangju 61203 (KR); CHA, Beom Seok, Gwangju 62044 (KR); KIM, Moonwon, Daejeon 34128 (KR); SHIN, Jun Won, Daejeon 34128 (KR)
(74) Representative: Ter Meer Steinmeister & Partner
(86) International application number: PCT/KR2023/015072
(87) International publication number: WO 2024/072123

(57) **Abstract**

An aerosol generation device according to an embodiment may comprise: a body which has a first surface, a second surface opposite to the first surface, and side surfaces between the first and second surfaces, and includes a mouthpiece end disposed at the first surface; an atomization region which is disposed within the body and connected to the mouthpiece end; a storage part which is connected to the atomization region and in which an aerosol-forming substrate is stored; and a surface wave generation part which is connected to the atomization region and generates surface waves, wherein: the surface wave generation part comprises a piezoelectric substrate extending to the atomization region and a converter disposed on the piezoelectric substrate; the converter converts an electrical signal into surface waves; the surface waves are provided to the atomization region; and the converter has a comb pattern shape.

## Description

### TECHNICAL FIELD

The disclosure relates to an aerosol generation device.

### BACKGROUND ART

Research on non-combustible tobacco products is in progress. An aerosol generation device generates aerosols by heating an aerosol-generating article.

The above description is information the inventor(s) acquired during the course of conceiving the present disclosure, or already possessed at the time, and is not necessarily art publicly known before the present application was filed.

### DISCLOSURE OF THE INVENTION

### TECHNICAL GOALS

An aspect of the present disclosure is to provide an aerosol generation device capable of generating fine aerosols of uniform size using surface waves.

An aspect of the present disclosure is to provide an aerosol generation device capable of non-thermal atomization of a highly viscous liquid.

An aspect of the present disclosure is to provide an aerosol generation device capable of realizing a rich amount of atomization of a highly viscous liquid.

An aspect of the present disclosure is to provide an aerosol generation device capable of visualizing the atomization of a highly viscous liquid.

### TECHNICAL SOLUTIONS

According to an embodiment, an aerosol generation device may include: a body including a first surface, a second surface opposite the first surface, and a side surface between the first surface and the second surface, and including a mouthpiece end disposed on the first surface; an atomization area disposed within the body and connected to the mouthpiece end; a storage part connected to the atomization area and storing therein an aerosol-forming substrate; and a surface wave generation part connected to the atomization area and generating surface waves. The surface wave generation part may include: a piezoelectric plate extending into the atomization area; and a transducer disposed on the piezoelectric plate. The transducer may be configured to convert an electrical signal into a surface wave. The surface wave may be provided to the atomization area, and the transducer may have a comb pattern shape.

According to an embodiment, an aerosol generation device may include: a body including a first surface, a second surface opposite the first surface, and a side surface between the first surface and the second surface, and including a mouthpiece end disposed on the first surface; an atomization area disposed within the body and connected to the mouthpiece end; a storage part connected to the atomization area and storing therein an aerosol-forming substrate; and a surface wave generation part connected to the atomization area and generating surface waves. The surface wave generation part may include: a piezoelectric plate extending into the atomization area; a transducer disposed on the piezoelectric plate; and a reflector disposed on an opposite side of the atomization area with the transducer therebetween. The transducer may be configured to convert an electrical signal into a surface wave. The surface wave may be provided to the atomization area, and the reflector may be configured to reflect the surface wave from the transducer into the atomization area.

### EFFECTS OF INVENTION

An aerosol generation device of various embodiments described herein may generate fine aerosols of uniform size using surface waves.

An aerosol generation device of various embodiments described herein may enable the non-thermal atomization of a highly viscous liquid.

An aerosol generation device of various embodiments described herein may realize a rich amount of atomization of a highly viscous liquid.

An aerosol generation device of various embodiments described herein may visualize the atomization of a highly viscous liquid.

The effects of the aerosol generation device according to embodiments are not limited to the foregoing effects, and other effects that are not described above may be clearly understood by a person of ordinary skill in the art from the following description.

### BRIEF DESCRIPTION OF DRAWINGS

FIG. 1 is a diagram illustrating an aerosol generation device according to an embodiment.
FIG. 2 is a diagram illustrating an aerosol generation device according to an embodiment.
FIG. 3 is a diagram illustrating an aerosol generation device according to an embodiment.
FIG. 4 is a diagram illustrating an aerosol generation device according to an embodiment.
FIG. 5 is a block diagram illustrating an aerosol generation device according to an embodiment.
FIG. 6 is a diagram illustrating an aerosol generation device according to an embodiment.
FIG. 7 is a diagram illustrating a surface wave generation part of an aerosol generation device according to an embodiment.
FIG. 8 is a diagram illustrating a transducer and a reflector of a surface wave generation part of an aerosol generation device according to an embodiment.
FIG. 9 is a schematic diagram illustrating a configuration of the transducer of FIG. 8.

### BEST MODE FOR CARRYING OUT THE INVENTION

The terms used in the embodiments described herein are selected from among common terms that are currently widely used, in consideration of their functions in the embodiments. However, the terms may be differently construed according to the intention of a person of ordinary skill in the art, precedents, or the advent of new technology. Also, in some particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that, when a certain part "includes" a certain component, the part does not exclude another component but may further include another component, unless the context clearly dictates otherwise. Also, terms such as "unit," "module," or the like as used in the disclosure may refer to a part for processing at least one function or operation and may be implemented as hardware, software, or a combination of hardware and software.

As used herein, an expression such as "at least one of" that precedes listed components modifies not each of the listed components but all the components. For example, the expression "at least one of a, b, or c" should be construed as including a, b, c, a and b, a and c, b and c, or a, b, and c.

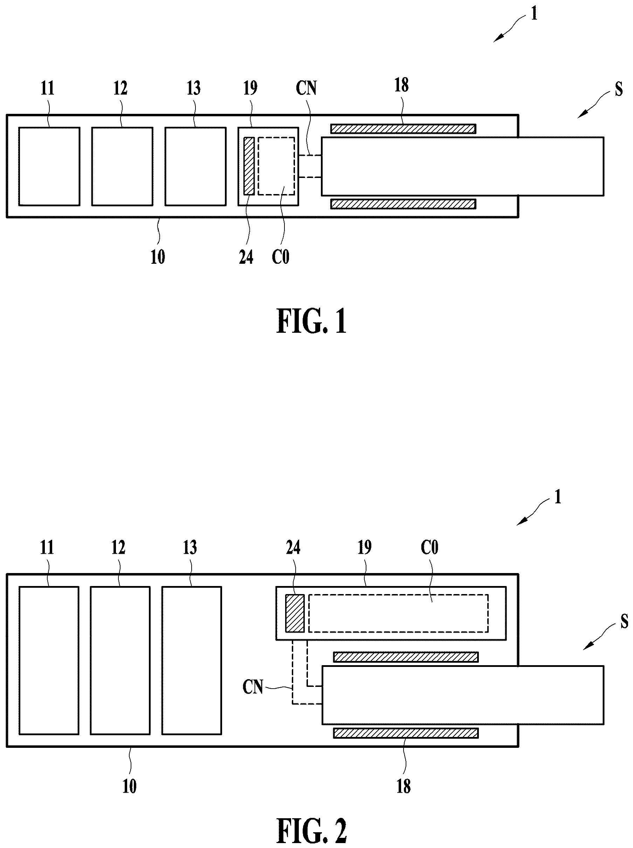

FIGS. 1 and 2 illustrate an aerosol generation device 1 according to various embodiments of the present disclosure.

Referring to FIG. 1, the aerosol generation device 1 may include at least one of a power source 11, a controller 12, a sensor 13, a heater 18, and a cartridge 19. At least one of the power source 11, the controller 12, the sensor 13, and the heater 18 may be disposed inside a body 10 of the aerosol generation device 1. The body 10 may provide an upwardly open space into which a stick S, an aerosol-generating article, is inserted. The upwardly open space may also be referred to herein as an insertion space. The insertion space may be formed by being recessed into the inside of the body 10 to a predetermined depth such that at least a portion of the stick S may be inserted therein. The depth of the insertion space may correspond to the length of an area containing an aerosol-generating material and/or medium in the stick S. A lower end of the stick S may be inserted into the inside of the body 10, and an upper end of the stick S may protrude outside the body 10. A user may inhale air with the externally exposed upper end of the stick S in the mouth.

The heater 18 may heat the stick S. The heater 18 may extend upwardly around the space in which the stick S is inserted. For example, the heater 18 may be provided in the form of a tube that includes a cavity therein. The heater 18 may be disposed around the insertion space. The heater 18 may be disposed to surround at least a portion of the insertion space. The heater 18 may heat the insertion space or the stick S inserted in the insertion space. The heater 18 may include an electrically resistive heater and/or an induction heating heater.

For example, the heater 18 may be a resistive heater. For example, the heater 18 may include an electrically conductive track, and the heater 18 may be heated as a current flows through the electrically conductive track. The heater 18 may be electrically coupled to the power source 11. The heater 18 may be heated directly by receiving a current from the power source 11.

For example, the aerosol generation device 1 may include an induction coil that surrounds the heater 18. The induction coil may cause the heater 18 to be heated. In this case, the heater 18 may be a susceptor, and the heater 18 may be heated by a magnetic field generated by an alternating current (AC) flowing through the induction coil. The magnetic field may pass through the heater 18 and generate an eddy current in the heater 18. The current may generate heat in the heater 18.

The stick S may include a susceptor inside the stick S, and the susceptor inside the stick S may be heated by the magnetic field generated by the AC current flowing through the induction coil.

The cartridge 19 may contain an aerosol-generating material in any one of a liquid state, a solid state, a gaseous state, and a gel state. The aerosol-generating material may contain a liquid composition. For example, the liquid composition may be a liquid containing a tobacco-containing substance including a volatile tobacco flavor component or a liquid containing a non-tobacco substance.

The cartridge 19 may be integrally formed with the body 10 or may be removably coupled to the body 10.

For example, referring to FIG. 1, the cartridge 19 may be integrally formed with the body 10 and may communicate with the insertion space via an airflow channel CN.

For example, referring to FIG. 2, a space may be formed on one side of the body 10, and at least a portion of the cartridge 19 may be inserted into the space formed on the one side of the body 10 such that the cartridge 19 is mounted to the body 10. The airflow channel CN may be defined by a portion of the cartridge 19 and/or a portion of the body 10, and the cartridge 19 may communicate with the insertion space via the airflow channel CN.

The body 10 may be formed in a structure that allows outside air to enter the inside of the body 10 with the cartridge 19 inserted. The outside air introduced into the body 10 may flow through the cartridge 19 and then into the oral cavity of the user.

The cartridge 19 may include a storage part C0 containing the aerosol-generating material and/or a heater 24 configured to heat the aerosol-generating material in the storage part C0. A liquid transfer means impregnated with (or containing) the aerosol-generating material may be disposed inside the storage part C0. In this case, the liquid transfer means may include a wick, such as, a cotton fiber, a ceramic fiber, a glass fiber, a porous ceramic, or the like. An electrically conductive track of the heater 24 may be formed in a structure of a coil that winds around the liquid transfer means or a structure in contact with one side of the liquid transfer means. The heater 24 may also be referred to herein as a cartridge heater 24.

The cartridge 19 may generate an aerosol. As the liquid transfer means is heated by the cartridge heater 24, the aerosol may be generated. The aerosol may be generated as the stick S is heated by the heater 18. As the aerosol generated by the cartridge heater 24 and the heater 18 passes through the stick S, a tobacco substance may be added to the aerosol, and the aerosol with the tobacco substance added may be inhaled through one end of the stick S into the oral cavity of the user.

The aerosol generation device 1 may include only the cartridge heater 24 but may not include the heater 18 in the body 10. In this case, a tobacco substance may be added to the aerosol generated by the cartridge heater 24 while the aerosol is passing through the stick S, and the aerosol with the tobacco substance added may be inhaled into the oral cavity of the user.

The aerosol generation device 1 may include a cap (not shown). The cap may be removably coupled to the body 10 to cover at least a portion of the cartridge 19 coupled to the body 10. The stick S may penetrate the cap to be inserted into the body 10.

The power source 11 may supply power to operate the components of the aerosol generation device 1. The power source 11 may be a battery. The power source 11 may supply power to at least one of the controller 12, the sensor 13, the cartridge heater 24, or the heater 18. In a case where the aerosol generation device 1 includes an induction coil, the power source 11 may supply power to the induction coil.

The controller 12 may control the overall operation of the aerosol generation device 1. The controller 12 may be mounted on a printed circuit board (PCB). The controller 12 may control the operation of at least one of the power source 11, the sensor 13, the heater 18, or the cartridge 19. The controller 12 may also control the operation of a display, a motor, or the like installed in the aerosol generation device 1. The controller 12 may check respective states of the components of the aerosol generation device 1 to determine whether the aerosol generation device 1 is in an operable state.

The controller 12 may analyze detection results of the sensor 13 and control further processing to be performed afterward. For example, the controller 12 may control the power to be supplied to the cartridge heater 24 and/or the heater 18 such that the operation of the cartridge heater 24 and/or the heater 18 is initiated or terminated based on the detection results of the sensor 13. For example, the controller 12 may control the amount of power supplied to the cartridge heater 24 and/or the heater 18 and the duration for which the power is supplied to allow the cartridge heater 24 and/or the heater 18 to be heated up to a predetermined temperature or maintained at an appropriate temperature, based on the detection results of the sensor 13.

The sensor 13 may include at least one of a temperature sensor, a puff sensor, an insertion detection sensor, a color sensor, a cartridge detection sensor, or a cap detection sensor. For example, the sensor 13 may sense at least one of a temperature of the heater 18, a temperature of the power source 11, or a temperature inside or outside the body 10. For example, the sensor 13 may sense a puff from the user. For example, the sensor 13 may sense whether the stick S is inserted in the insertion space. For example, the sensor 13 may sense whether the cartridge 19 is loaded. For example, the sensor 13 may sense whether the cap is mounted.

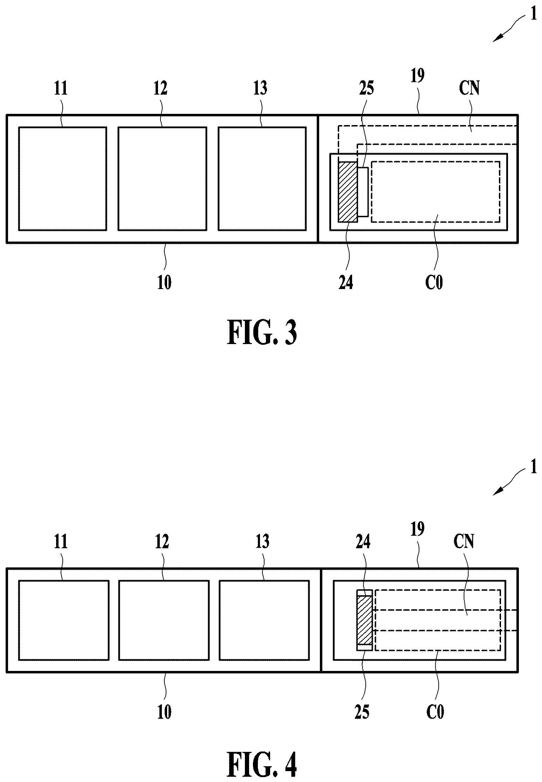

FIGS. 3 and 4 illustrate an aerosol generation device 1 according to various embodiments of the present disclosure.

Referring to FIGS. 3 and 4, the aerosol generation device 1 may include a body 10 and a cartridge 19. The aerosol generation device 10 may include at least one of a power source 11, a controller 12, and a sensor 13. At least one of the power source 11, the controller 12, or the sensor 13 may be disposed inside the body 10. In the body 10, the cartridge 19 that is an aerosol-generating article may be provided. A user may inhale an aerosol with a mouthpiece provided at one end of the cartridge 19 in the mouth.

The cartridge 19 may contain, in an internal chamber C0, an aerosol-generating material in any one of a liquid state, a solid state, a gaseous state, and a gel state. The aerosol-generating material may include a liquid composition. For example, the liquid composition may be a liquid containing a tobacco-containing substance including a volatile tobacco flavor component or a liquid containing a non-tobacco substance.

The cartridge 19 may be removably coupled to the body 10. The cartridge 19 may be mounted to the body 10 as it is inserted into the body 10.

The body 10 may be formed in a structure that allows outside air to enter the inside of the body 10 with the cartridge 19 inserted. The outside air introduced into the body 10 may flow through the cartridge 19 and then into the oral cavity of the user via an airflow channel CN.

The cartridge 19 may include the chamber C0 containing the aerosol-generating material and/or a heater 24 configured to heat the aerosol-generating material in the chamber C0. A liquid transfer means 25 impregnated with the aerosol-generating material may be disposed inside the chamber C0. In this case, the liquid transfer means 25 may include a wick, such as, a cotton fiber, a ceramic fiber, a glass fiber, a porous ceramic, or the like. An electrically conductive track of the heater 24 may be formed in a structure of a coil that winds around the liquid transfer means 25 or a structure in contact with one side of the liquid transfer means 25. The heater 24 may also be referred to herein as a cartridge heater.

The cartridge 19 may generate an aerosol. As the liquid transfer means 25 is heated by the cartridge heater 24, the aerosol may be generated. The generated aerosol may be inhaled through the airflow channel CN into the oral cavity of the user.

The airflow channel CN may be provided in the cartridge 19. The airflow channel CN may allow a chamber C1 in which the heater 24 of the cartridge 19 is disposed and the outside of the cartridge 19 to communicate. One end of the airflow channel CN may open into the chamber C1 in which the heater 24 is disposed and the other end thereof may communicate with the mouthpiece. For example, referring to FIG. 3, the airflow channel CN may extend from one side of the chamber C0 of the cartridge 19 along a longitudinal direction of the cartridge 19. For example, referring to FIG. 4, the airflow channel CN may extend along the longitudinal direction of the cartridge 19 by passing through the chamber C0 of the cartridge 19.

The power source 11 may supply power to operate the components of the aerosol generation device 1. The power source 11 may be a battery. The power source 11 may supply power to at least one of the controller 12, the sensor 13, or the cartridge heater 24.

The controller 12 may control the overall operation of the aerosol generation device 1. The controller 12 may be mounted on a PCB. The controller 12 may control the operation of at least one of the power source 11, the sensor 13, or the cartridge 19. The controller 12 may also control the operation of a display, a motor, or the like installed in the aerosol generation device 1. The controller 12 may check respective states of the components of the aerosol generation device 1 to determine whether the aerosol generation device 1 is in an operable state.

The controller 12 may analyze detection results of the sensor 13 and control further processing to be performed afterward. For example, the controller 12 may control the power to be supplied to the cartridge heater 24 such that the operation of the cartridge heater 24 is initiated or terminated based on the detection results of the sensor 13. For example, the controller 12 may control the amount of power supplied to the cartridge heater 24 and the duration for which the power is supplied to allow the cartridge heater 24 to be heated up to a predetermined temperature or maintained at an appropriate temperature, based on the detection results of the sensor 13.

The sensor 13 may include at least one of a temperature sensor, a puff sensor, a cartridge detection sensor, or a motion detection sensor. For example, the sensor 13 may sense at least one of a temperature of the cartridge heater 24, a temperature of the power source 11, or a temperature inside or outside the body 10. For example, the sensor 13 may sense a puff from the user. For example, the sensor 13 may sense whether the cartridge 19 is loaded. For example, the sensor 13 may sense a movement of the aerosol generation device 1.

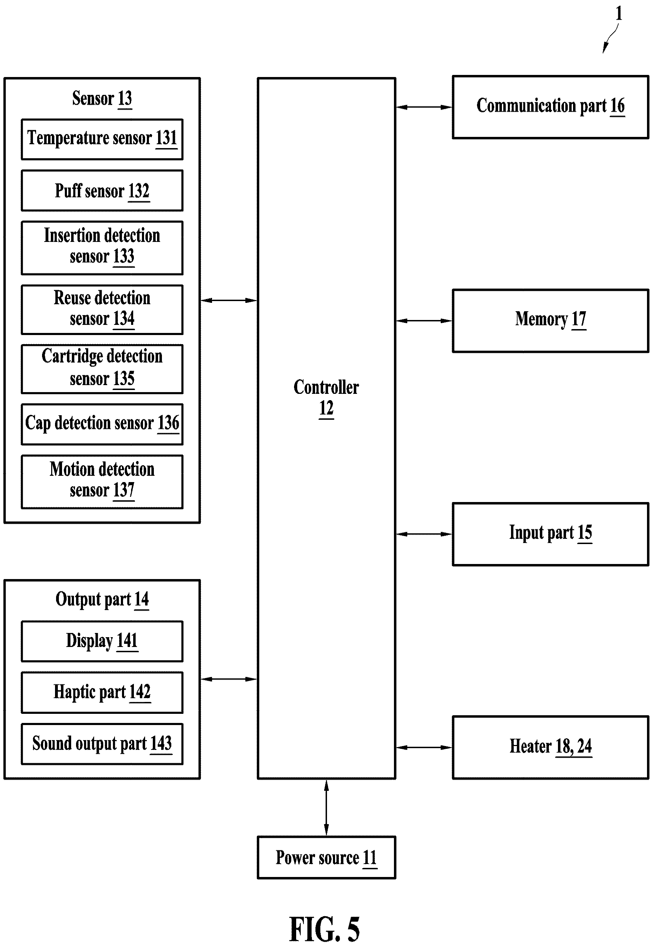

FIG. 5 is a block diagram illustrating an aerosol generation device 1 according to an embodiment.

The aerosol generation device 1 may include a power source 11, a controller 12, a sensor 13, an output part 14, an input part 15, a communication part 16, a memory 17, and at least one heater 18, 24. However, the internal structure of the aerosol generation device 1 is not limited to what is shown in FIG. 5. That is, depending on the design of the aerosol generation device 1, some of the components shown in FIG. 5 may be omitted, or some new components may be added, as will be understood by a person of ordinary skill in the art to which the present disclosure pertains.

The sensor 13 may sense a state or condition of the aerosol generation device 1 or a state or condition around the aerosol generation device 1 and transmit detected information to the controller 12. Based on the detected information, the controller 12 may control the aerosol generation device 1 to perform various functions, such as, controlling the operation of the cartridge heater 24 and/or the heater 18, restricting smoking, determining whether a stick S and/or the cartridge 19 is inserted, displaying a notification, or the like.

The sensor 13 may include at least one of a temperature sensor 131, a puff sensor 132, an insertion detection sensor 133, a reuse detection sensor 134, a cartridge detection sensor 135, a cap detection sensor 136, or a motion detection sensor 137.

The temperature sensor 131 may sense a temperature at which the cartridge heater 24 and/or the heater 18 is heated. The aerosol generation device 1 may include a separate temperature sensor configured to sense the temperature of the cartridge heater 24 and/or the heater 18, or the cartridge heater 24 and/or the heater 18 itself may act as a temperature sensor.

The temperature sensor 131 may output a signal corresponding to the temperature of the cartridge heater 24 and/or the heater 18. For example, the temperature sensor 131 may include a resistor element whose resistance value changes in response to a change in the temperature of the cartridge heater 24 and/or the heater 18. For example, this may be implemented by a thermistor, a device that uses the property of changing resistance with temperature. In this case, the temperature sensor 131 may output a signal corresponding to a resistance value of the resistor element as the signal corresponding to the temperature of the cartridge heater 24 and/or the heater 18. For example, the temperature sensor 131 may include a sensor configured to detect a resistance value of the cartridge heater 24 and/or the heater 18. In this case, the temperature sensor 131 may output a signal corresponding to a resistance value of the cartridge heater 24 and/or the heater 18 as the signal corresponding to the temperature of the cartridge heater 24 and/or the heater 18.

The temperature sensor 131 may be disposed around the power source 11 to monitor a temperature of the power source 11. The temperature sensor 131 may be disposed near the power source 11. For example, the temperature sensor 131 may be attached to one surface of a battery, which is the power source 11. For example, the temperature sensor 131 may be mounted on one surface of a PCB.

The temperature sensor 131 may be disposed inside the body 10 to sense an internal temperature of the body 10.

The puff sensor 132 may sense a puff from the user based on various physical changes in an airflow path. The puff sensor 132 may output a signal corresponding to the puff. For example, the puff sensor 132 may be a pressure sensor. The puff sensor 132 may output a signal corresponding to an internal pressure of the aerosol generation device 1. In this case, the internal pressure of the aerosol generation device 1 may correspond to a pressure of the airflow path through which gas flows. The puff sensor 132 may be disposed at a position in the aerosol generation device 1 corresponding to the airflow path through which the gas flows.

The insertion detection sensor 133 may detect insertion and/or removal of the stick S. The insertion detection sensor 133 may detect a signal change as the stick S is inserted and/or removed. The insertion detection sensor 133 may be installed around the insertion space. The insertion detection sensor 133 may detect the insertion and/or removal of the stick S based on a change in permittivity inside the insertion space. For example, the insertion detection sensor 133 may be an inductive sensor and/or a capacitive sensor.

The inductive sensor may include at least one coil. The coil of the inductive sensor may be disposed near the insertion space. For example, in a case where a magnetic field changes in the vicinity of the coil through which a current flows, the properties of the current flowing in the coil may change according to Faraday's law of electromagnetic induction. The properties of the current flowing in the coil may include an AC frequency, a current value, a voltage value, an inductance value, an impedance value, or the like.

The inductive sensor may output a signal corresponding to the properties of the current flowing in the coil. For example, the inductive sensor may output a signal corresponding to the inductance value of the coil.

The capacitive sensor may include a conductor. The conductor of the capacitive sensor may be disposed near the insertion space. The capacitive sensor may output a signal corresponding to an electromagnetic property of the surroundings, such as, for example, a capacitance around the conductor. For example, in a case where the stick S including a metallic wrapper is inserted in the insertion space, the electromagnetic property around the conductor may be changed by the wrapper of the stick S.

The reuse detection sensor 134 may detect whether the stick S has been reused. The reuse detection sensor 134 may be a color sensor. The color sensor may sense a color of the stick S. The color sensor may sense a color of a portion of a wrapper that wraps the outside of the stick S. The color sensor may detect, based on light reflected from an object, a value for an optical property corresponding to a color of the object. For example, the optical property may be a wavelength of light. The color sensor may be implemented as one component with a proximity sensor or may be implemented as a separate component from the proximity sensor.

At least a portion of the wrapper included in the stick S may be changed in color by an aerosol. The reuse detection sensor 134 may be disposed at a position at which the portion of the wrapper with the color changing by the aerosol is disposed, when the stick S is inserted in the insertion space. For example, the color of the portion of the wrapper may be a first color before the stick S is used by the user, but may be changed to a second color as the portion of the wrapper is wet by the aerosol while the aerosol generated by the aerosol generation device 1 is passing through the stick S. Alternatively, the color of the portion of the wrapper may remain the second color after being changed from the first color to the second color.

The cartridge detection sensor 135 may detect insertion and/or removal of the cartridge 19. The cartridge detection sensor 135 may be implemented as an inductive sensor, a capacitive sensor, a resistive sensor, a Hall sensor (e.g., a Hall integrated circuit (IC)) using a Hall effect, or the like.

The cap detection sensor 136 may detect attachment and/or removal of the cap. In a case where the cap is removed from the body 10, the cartridge 19 and a portion of the body 10 that have been covered by the cap may be exposed to the outside. The cap detection sensor 136 may be implemented as a contact sensor, a Hall sensor (e.g., a Hall IC), an optical sensor, or the like.

The motion detection sensor 137 may detect a movement of the aerosol generation device 1. The motion detection sensor 137 may be implemented as at least one of an acceleration sensor or a gyro sensor.

In addition to the sensors 131 to 137 described above, the sensor 13 may further include at least one of a humidity sensor, a barometric pressure sensor, a magnetic sensor, a geomagnetic sensor, a position sensor (e.g., a global positioning system (GPS)), or a proximity sensor. The functions of the respective sensors may be intuitively inferred by a person of ordinary skill in the art from their designations, and thus detailed descriptions thereof are omitted here.

The output part 14 may output information about a state of the aerosol generation device 1 and provide the information to the user. The output part 14 may include, but is not limited to, at least one of a display 141, a haptic part 142, and a sound output part 143. For example, in a case where the display 141 and a touchpad are layered to form a touchscreen, the display 141 may be used as an input device in addition to an output device.

The display 141 may visually provide the information about the aerosol generation device 1 to the user. For example, the information about the aerosol generation device 1 may include various information, such as, for example, a charging/discharging state of the power source 11 of the aerosol generation device 1, a pre-heating state of the heater 18, an insertion/removal state of the stick S and/or the cartridge 19, an attachment/removal state of the cap, or a condition in which the use of the aerosol generation device 1 is restricted (e.g., when an abnormal item is detected), and the display 141 may output the information externally. For example, the display 141 may be provided in the form of a light-emitting diode (LED) element. For example, the display 141 may be a liquid-crystal display (LCD) panel, an organic light-emitting diode (OLED) display panel, or the like.

The haptic part 142 may convert an electrical signal into a mechanical or electrical stimulus to tactilely provide the information about the aerosol generation device 1 to the user. For example, when initial power is supplied to the cartridge heater 24 and/or the heater 18 for a set period of time, the haptic part 142 may generate a vibration corresponding to the completion of initial pre-heating. The haptic part 142 may include a vibration motor, a piezoelectric element, or an electrical stimulation device.

The sound output part 143 may audibly provide the information about the aerosol generation device 1 to the user. For example, the sound output part 143 may convert an electrical signal into an acoustic signal and output it to the outside.

The power source 11 may supply power to be used to operate the aerosol generation device 1. The power source 11 may supply power to the cartridge heater 24 and/or the heater 18 such that they are heated therewith. The power source 11 may also supply power required for operations of other components within the aerosol generation device 1, such as, the sensor 13, the output part 14, the input part 15, the communication part 16, and the memory 17. The power source 11 may be a rechargeable battery or a disposable battery. For example, the power source 11 may be, but is not limited to, a lithium polymer (LiPoly) battery.

Although not shown in FIG. 5, the aerosol generation device 1 may further include a power protection circuit. The power protection circuit may be electrically connected to the power source 11 and may include a switching element.

The power protection circuit may interrupt a power path leading to the power source 11 in response to a predetermined condition. For example, the power protection circuit may disconnect the power path to the power source 11 in response to a voltage level of the power source 11 being greater than or equal to a first voltage corresponding to an overcharge. For example, the power protection circuit may disconnect the power path to the power source 11 in response to the voltage level of the power source 11 being less than a second voltage corresponding to an overdischarge.

The heater 18 may receive power from the power source 11 to heat a medium or an aerosol-generating material in the stick S. Although not shown in FIG. 5, the aerosol generation device 1 may further include a power conversion circuit (e.g., a direct current (DC) to DC (DC/DC) converter) that converts power from the power source 11 and provide it to the cartridge heater 24 and/or the heater 18. Further, in a case where the aerosol generation device 1 generates an aerosol by induction heating, the aerosol generation device 1 may further include a DC to AC (DC/AC) converter that converts DC power of the power source 11 into AC power.

The controller 12, the sensor 13, the output part 14, the input part 15, the communication part 16, and the memory 17 may receive power from the power source 11 to perform their functions. Although not shown in FIG. 1, a power conversion circuit, such as, a low-dropout (LDO) circuit or a voltage regulator circuit, may be further included to convert and supply power from the power source 11 to the respective components. In addition, although not shown in FIG. 5, a noise filter may be provided between the power source 11 and the heater 18. The noise filter may be a low-pass filter (LPF). The LPF may include at least one inductor and capacitor. A cutoff frequency of the LPF may correspond to a frequency of a high-frequency switching current applied from the power source 11 to the heater 18. The LPF may prevent a high-frequency noise component from being applied to the sensor 13, for example, the insertion detection sensor 133.

In one embodiment, the cartridge heater 24 and/or the heater 18 may be formed of any suitable electrically resistive material. For example, the electrically resistive material may be a metal or a metal alloy including, but not limited to, titanium, zirconium, tantalum, platinum, nickel, cobalt, chromium, hafnium, niobium, molybdenum, tungsten, tin, gallium, manganese, iron, copper, stainless steel, nichrome, or the like. The heater 18 may be implemented as, as non-limiting examples, a metal heating wire, a metal heating plate with an electrically conductive track disposed thereon, a ceramic heating element, or the like.

In another embodiment, the heater 18 may be an induction heating heater. For example, the heater 18 may include a susceptor that is heated via a magnetic field applied by a coil, thereby heating the aerosol-generating material.

The input part 15 may receive information input from the user and/or output information to the user. For example, the input part 15 may be a touch panel. The touch panel may include at least one touch sensor that detects touch. For example, the touch sensor may include, but is not limited to, a capacitive touch sensor, a resistive touch sensor, a surface acoustic wave touch sensor, an infrared touch sensor, or the like.

The display 141 and the touch panel may be implemented as a single panel. For example, the touch panel may be embedded in the display 141, for example, in an on-cell type or in-cell type. For example, the touch panel may be an add-on type one provided on the display 141.

The input part 15 may include, but is not limited to, a button, a keypad, a dome switch, a jog wheel, a jog switch, or the like.

The memory 17, which is hardware that stores various data processed in the aerosol generation device 1, may store data processed by the controller 12 and data to be processed. The memory 17 may include a storage medium of at least one type of a flash memory type, a hard disk type, a multimedia card micro type, a card type (e.g., secure digital (SD) or extreme digital (XD) memory, etc.), a random-access memory (RAM), a static random-access memory (SRAM), a read-only memory (ROM), an electrically erasable programmable read-only memory (EEPROM), a programmable read-only memory (PROM), a magnetic memory, magnetic disk, and optical disc. The memory 17 may store data on an operating time of the aerosol generation device 1, a maximum number of puffs, a current number of puffs, at least one temperature profile, and a smoking pattern of the user.

The communication part 16 may include at least one component for communicating with other electronic devices. For example, the communication part 16 may include at least one of a short-range communication unit and a wireless communication unit.

The short-range wireless communication unit may include, but is not limited to, a Bluetooth communication unit, a Bluetooth low energy (BLE) communication unit, a near-field communication (NFC) unit, a wireless local-area network (WLAN) communication (e.g., (Wi-Fi) unit, a Zigbee communication unit, an infrared data association (IrDA) communication unit, a Wi-Fi direct (WFD) communication unit, an ultra-wideband (UWB) communication unit, an Ant+ communication unit, or the like.

The wireless communication unit may include, but is not limited to, a cellular network communication unit, an Internet communication unit, a computer network (e.g., local area network (LAN) or wide area network (WAN)) communication unit, or the like.

Although not shown in FIG. 5, the aerosol generation device 1 may further include a connection interface, such as, a universal serial bus (USB) interface, and may be connected to other external devices via the connection interface (e.g., a USB interface) to transmit and receive information or to charge the power source 11.

The controller 12 may control the overall operation of the aerosol generation device 1. In one embodiment, the controller 12 may include at least one processor. The processor may be implemented as an array of multiple logic gates or as a combination of a general-purpose microprocessor and a memory storing a program to be executed on the microprocessor. It will be understood by a person of ordinary skill in the art to which the present disclosure pertains that it may also be implemented in other forms of hardware.

The controller 12 may control a temperature of the heater 18 by controlling the power supply from the power source 11 to the heater 18. The controller 12 may control a temperature of the cartridge heater 24 and/or the heater 18 based on a temperature of the cartridge heater 24 and/or the heater 18 sensed by the temperature sensor 131. Based on the temperature of the cartridge heater 24 and/or the heater 18, the controller 12 may regulate the power to be supplied to the cartridge heater 24 and/or the heater 18. For example, the controller 12 may determine a target temperature for the cartridge heater 24 and/or the heater 18 based on a temperature profile stored in the memory 17.

The aerosol generation device 1 may include a power supply circuit (not shown) electrically connected to the power source 11 between the power source 11 and the cartridge heater 24 and/or the heater 18. The power supply circuit may be electrically connected to the cartridge heater 24, the heater 18, or an induction coil 181. The power supply circuit may include at least one switching element. The switching element may be implemented by a bipolar junction transistor (BJT), a field-effective transistor (FET), or the like. The controller 12 may control the power supply circuit.

The controller 12 may control the power supply by controlling switching of the switching element of the power supply circuit. The power supply circuit may be an inverter that converts DC power output from the power source 11 into AC power. For example, the inverter may be configured as a full-bridge or half-bridge circuit including a plurality of switching elements.

The controller 12 may turn on the switching element to supply power to the cartridge heater 24 and/or the heater 18 from the power source 11. The controller 12 may turn off the switching element to cut off the power supply to the cartridge heater 24 and/or the heater 18. The controller 12 may regulate a current supplied from the power source 11 by regulating a frequency and/or duty ratio of a current pulse input to the switching element.

The controller 12 may control a voltage output from the power source 11 by controlling switching of the switching element of the power supply circuit. The power conversion circuit may convert the voltage output from the power source 11. For example, the power conversion circuit may include a buck converter that steps down the voltage output from the power source 11. For example, the power conversion circuit may be implemented through a buck-boost converter, a Zener diode, or the like.

The controller 12 may control the on/off operation of the switching element included in the power conversion circuit to regulate the level of a voltage output from the power conversion circuit. For example, when the switching element is continuously in an "on" state, the level of a voltage output from the power conversion circuit may correspond to the level of a voltage output from the power source 11. A duty ratio for the on/off operation of the switching element may correspond to a ratio of the voltage output from the power conversion circuit to the voltage output from the power source 11. As the duty ratio for the on/off operation of the switching element decreases, the level of the voltage output from the power conversion circuit may decrease. The heater 18 may be heated based on the voltage output from the power conversion circuit.

The controller 12 may control the power supply to the heater 18 using at least one of a pulse-width modulation (PWM) method or a proportional-integral-differential (PID) method.

For example, using the PWM method, the controller 12 may control a current pulse having a predetermined frequency and duty ratio to be supplied to the heater 18. In this case, by regulating the frequency and duty ratio of the current pulse, the controller 12 may control the power to be supplied to the heater 18.

For example, the controller 12 may determine a target temperature that is a target to be controlled, based on a temperature profile. The controller 12 may control the power to be supplied to the heater 18, using the PID method, which is a feedback control method based on a difference value between the temperature of the heater 18 and the target temperature, a value acquired by integrating the difference value over time, and a value acquired by differentiating the difference value over time.

The controller 12 may prevent the cartridge heater 24 and/or the heater 18 from being overheated. For example, the controller 12 may control the operation of the power conversion circuit such that the power supply to the cartridge heater 24 and/or the heater 18 is interrupted based on the temperature of the cartridge heater 24 and/or the heater 18 exceeding a predetermined limit temperature. For example, in response to the temperature of the cartridge heater 24 and/or the heater 18 exceeding the predetermined limit temperature, the controller 12 may reduce the amount of power to be supplied to the cartridge heater 24 and/or the heater 18 by a certain percentage. For example, in response to the temperature of the cartridge heater 24 exceeding the predetermined limit temperature, the controller 12 may determine that the aerosol-generating material contained in the cartridge 19 is exhausted and may cut off the power supply to the cartridge heater 24.

The controller 12 may control charging and discharging of the power source 11. The controller 12 may check the temperature of the power source 11 based on an output signal of the temperature sensor 131.

In a case where a power line is connected to a battery terminal of the aerosol generation device 1, the controller 12 may determine whether the temperature of the power source 11 is greater than or equal to a first limit temperature, which is a criterion for cutting off charging the power source 11. In response to the temperature of the power source 11 being less than the first limit temperature, the controller 12 may control the power source 11 to be charged based on a preset charging current. In response to the temperature of the power source 11 being greater than or equal to the first limit temperature, the controller 12 may cut off charging the power source 11.

While the aerosol generation device 1 is powered on, the controller 12 may determine whether the temperature of the power source 11 is greater than or equal to a second limit temperature, which is a criterion for cutting off discharging the power source 11. In response to the temperature of the power source 11 being less than the second limit temperature, the controller 12 may control the power stored in the power source 11 to be used. In response to the temperature of the power source 11 being greater than or equal to the second limit temperature, the controller 12 may stop using the power stored in the power source 11.

The controller 12 may calculate a residual capacity of the power stored in the power source 11. For example, the controller 12 may calculate the residual capacity of the power source 11 based on sensed voltage and/or current values of the power source 11.

The controller 12 may determine, via the insertion detection sensor 133, whether the stick S is inserted in the insertion space. The controller 12 may determine that the stick S is inserted based on an output signal of the insertion detection sensor 133. In response to a determination that the stick S is inserted in the insertion space, the controller 12 may control the power supply to the cartridge heater 24 and/or the heater 18 such that power is supplied thereto. For example, the controller 12 may supply power to the cartridge heater 24 and/or the heater 18 based on the temperature profile stored in the memory 17.

The controller 12 may determine whether the stick S is removed from the insertion space. For example, the controller 12 may determine, via the insertion detection sensor 133, whether the stick S is removed from the insertion space. For example, in response to the temperature of the heater 18 being greater than or equal to a limit temperature or in response to a temperature change slope of the heater 18 being greater than or equal to a set slope, the controller 12 may determine that the stick S is removed from the insertion space. In response to a determination that the stick S is removed from the insertion space, the controller 12 may cut off the power supply to the cartridge heater 24 and/or the heater 18 such that the power supplied thereto is cut off.

The controller 12 may control a power supply time and/or amount of the heater 18, based on a state of the stick S sensed by the sensor 13. The controller 12 may determine, based on a lookup table, a level range in which a level of a signal from a capacitance sensor is included. Based on the determined level range, the controller 12 may determine a moisture amount of the stick S.

In a case where the stick S is in an overwetted condition, the controller 12 may control the power supply time for which the power is supplied to the heater 18 to increase a pre-heating time of the stick S compared to in a normal condition.

The controller 12 may determine, via the reuse detection sensor 134, whether the stick S inserted in the insertion space is reused. For example, the controller 12 may compare a sensed value of a signal from the reuse detection sensor 134 to a first reference range in which the first color is included and, in response to the sensed value being included in the first reference range, determine that the stick S has not been used. For example, the controller 12 may compare the sensed value of the signal from the reuse detection sensor 134 to a second reference range in which the second color is included and, in response to the sensed value being included in the second reference range, determine that the stick S has been used. In response to a determination that the stick S has been used, the controller 12 may cut off the power supply to the cartridge heater 24 and/or the heater 18.

The controller 12 may determine, via the cartridge detection sensor 135, whether the cartridge 19 is coupled and/or removed. For example, the controller 12 may determine whether the cartridge 19 is coupled and/or removed based on a sensed value of a signal from the cartridge detection sensor 135.

The controller 12 may determine whether the aerosol-generating material in the cartridge 19 is exhausted. For example, the controller 12 may apply power to pre-heat the cartridge heater 24 and/or the heater 18, and determine whether a temperature of the cartridge heater 24 exceeds a limit temperature during a pre-heating period. In response to the temperature of the cartridge heater 24 exceeding the limit temperature, the controller 12 may determine that the aerosol-generating material in the cartridge 19 is exhausted. In response to a determination that the aerosol-generating material in the cartridge 19 is exhausted, the controller 12 may cut off the power supply to the cartridge heater 24 and/or the heater 18.

The controller 12 may determine whether the cartridge 19 is available for use. For example, based on data stored in the memory 17, in response to a current number of puffs being greater than or equal to a maximum number of puffs set for the cartridge 19, the controller 12 may determine that the cartridge 19 is not available for use. For example, when a total amount of time for which the heater 24 is heated is greater than or equal to a preset maximum amount of time, or when a total amount of power supplied to the heater 24 is greater than or equal to a preset maximum amount of power, the controller 12 may determine that the cartridge 19 is not available for use.

The controller 12 may determine, via the puff sensor 132, inhalation of the user. For example, the controller 12 may determine whether a puff is generated based on a sensed value of a signal from the puff sensor 132. For example, the controller 12 may determine the intensity of the puff based on the sensed value of the signal from the puff sensor 132. In response to the number of puffs reaching a preset maximum number of puffs, or in response to no puff being detected for a preset period of time or more, the controller 12 may cut off the power supply to the cartridge heater 24 and/or the heater 18.

The controller 12 may determine, via the cap detection sensor 136, whether the cap is coupled and/or removed. For example, the controller 12 may determine whether the cap is coupled and/or removed based on a sensed value of a signal from the cap detection sensor 136.

The controller 12 may control the output part 14 based on detection results of the sensor 13. For example, when the number of puffs counted via the puff sensor 132 reaches a preset number, the controller 12 may notify the user that the aerosol generation device 1 is about to end, via at least one of the display 141, the haptic part 142, or the sound output part 143. For example, the controller 12 may notify the user via the output part 14 based on a determination that no stick S is present in the insertion space. For example, the controller 12 may notify the user via the output part 14 based on a determination that the cartridge 19 and/or cap is not loaded. For example, the controller 12 may transmit information about the temperature of the cartridge heater 24 and/or the heater 18 to the user via the output part 14.

The controller 12 may store, in the memory 17, a history of an event that has occurred based on the occurrence of predetermined events, and update the history. The events may include, for example, detection of insertion of the stick S, initiation of heating of the stick S, detection of a puff, termination of a puff, detection of overheating of the cartridge heater 24 and/or the heater 18, detection of an overvoltage application to the cartridge heater 24 and/or the heater 18, termination of heating of the stick S, power on/off operation of the aerosol generation device 1, initiation of charging of the power source 11, detection of overcharging of the power source 11, termination of charging of the power source 11, or the like, which are performed by the aerosol generation device 1. The history of the event may include a time when the event has occurred, log data corresponding to the event, or the like. For example, in a case where a predetermined event is the detection of insertion of the stick S, the log data corresponding to the event may include data on a sensing value or the like of the insertion detection sensor 133. For example, in a case where a predetermined event is detection of overheating of the cartridge heater 24 and/or the heater 18, the log data corresponding to the event may include data on a temperature of the cartridge heater 24 and/or the heater 18, a voltage applied to the cartridge heater 24 and/or the heater 18, a current flowing through the cartridge heater 24 and/or the heater 18, or the like.

The controller 12 may control to form a communication link with an external device, such as, a mobile terminal of the user. Upon receiving authentication-related data from the external device via the communication link, the controller 12 may lift a restriction on the use of at least one function of the aerosol generation device 1. In this case, the authentication-related data may include data indicative of the completion of user authentication for a user corresponding to the external device. The user may perform the user authentication via the external device. The external device may determine whether user data is valid based on the birthday of the user, a unique number indicating the user, or the like, and may receive data on an authorization of the user for using the aerosol generation device 1 from an external server. Based on the data on the authorization for use, the external device may transmit the data indicating the completion of the user authentication to the aerosol generation device 1. When the user authorization is completed, the controller 12 may lift the restriction on the use of the at least one function of the aerosol generation device 1. For example, when the user authorization is completed, the controller 12 may lift a restriction on the use of a heating function to supply power to the heater 18.

The controller 12 may transmit data on a state of the aerosol generation device 1 to the external device via the communication link formed with the external device. Based on the received data on the state, the external device may output a residue capacity, an operation mode, or the like of the power source 11 of the aerosol generation device 1, via a display of the external device.

The external device may transmit a position search request to the aerosol generation device 1 based on an input that initiates a search for a position of the aerosol generation device 1. Upon receiving the position search request from the external device, the controller 12 may control at least one output device to perform an operation corresponding to the search for the position based on the received position search request. For example, in response to the position search request, the haptic part 142 may generate a vibration. For example, in response to the position search request, the display 141 may output an object corresponding to the search for the position and the termination of the search.

Upon receiving firmware data from the external device, the controller 12 may control a firmware update to be performed. The external device may determine a current version of firmware of the aerosol generation device 1 and determine whether a new version of firmware exists. In response to an input requesting a firmware download being received, the external device may receive firmware data of the new version of firmware and may transmit the firmware data of the new version of firmware to the aerosol generation device 1. In response to the firmware data of the new version of firmware being received, the controller 12 may control the firmware update of the aerosol generation device 1 to be performed.

The controller 12 may transmit data on sensing values of the sensor 13 to an external server (not shown) via the communication part 16, and may receive, from the server, and store a learning model generated by learning the sensing values through machine learning, such as, deep learning. The controller 12 may use the learning model received from the server to perform operations, such as, for example, determining an inhalation pattern of the user, generating a temperature profile, or the like. The controller 12 may store, in the memory 17, the data on the sensing values of the sensor 13 and data for training an artificial neural network (ANN). For example, the memory 17 may store a database (DB) for each component of the aerosol generation device 1, and weights and bias of an architecture of the ANN to train the ANN. The controller 12 may learn the data on sensing values of the sensor 13, the inhalation pattern of the user, the temperature profile, or the like, which are stored in the memory 17, to generate at least one learning model to be used to determine the inhalation pattern of the user, generate the temperature profile, or the like.

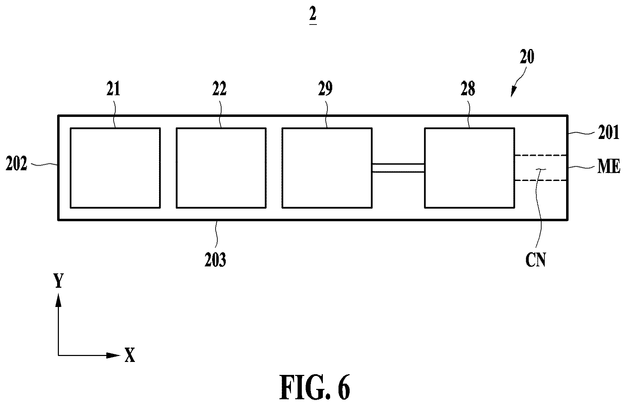

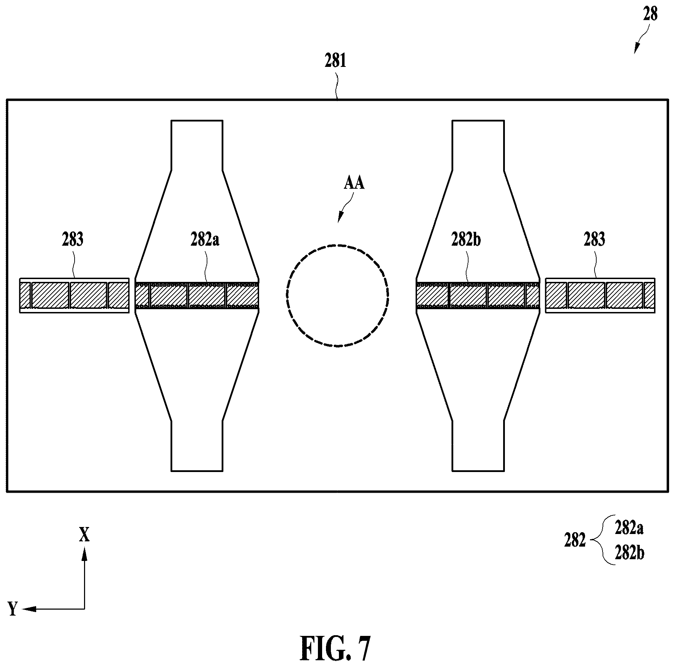

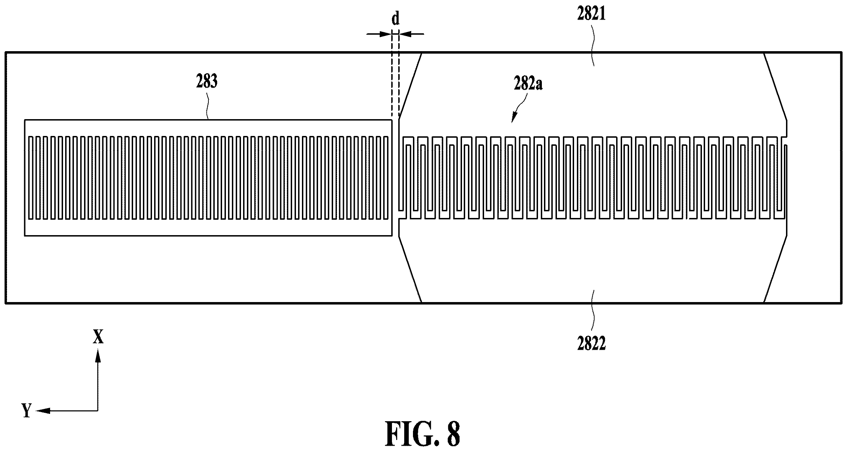

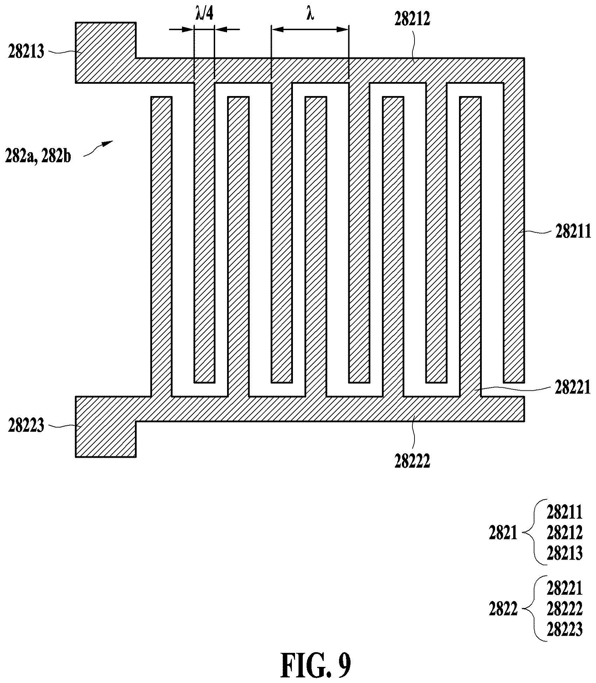

FIG. 6 is a diagram illustrating an aerosol generation device 2 according to an embodiment, and FIG. 7 is a diagram illustrating a surface wave generation part 28 of the aerosol generation device 2 according to an embodiment. FIG. 8 is a diagram illustrating a transducer 282a and a reflector 283 of the surface wave generation part 28 of the aerosol generation device 2 according to an embodiment, and FIG. 9 is a schematic diagram illustrating a configuration of the transducer of FIG. 8.

Referring to FIG. 6, the aerosol generation device 2 of one embodiment may aerosolize a liquid stored in a storage part 29 using surface acoustic waves (or surface waves herein). The aerosol generation device 2 may include a body 20, a power source 21, a controller 22, the surface wave generation part 28, and the storage part 29.

In one embodiment, the body 20 may include a first surface 201, a second surface 202 on an opposite side of the first surface 201, and a side surface 203 between the first surface 201 and the second surface 202. On the first surface 201 of the body 20, a mouthpiece end ME may be provided. The mouthpiece end ME may be used by a user to inhale an aerosol. In an example, the mouthpiece end ME may be a portion that comes into contact with an oral region of the user, and the aerosol may travel to the user through the mouthpiece end ME via an airflow channel CN. In another example, the mouthpiece end ME may be included in an internal space (e.g., an insertion space) into which an aerosol-generating article (e.g., the stick S of FIG. 1 or 2) is inserted. The internal space may be formed by being recessed a predetermined depth toward the inside of the body 20 (e.g., toward a -X direction in FIG. 6) to allow the aerosol-generating article to be inserted therein. The depth of the internal space may correspond to a length of an area containing an aerosol-generating material and/or medium in the aerosol-generating article. An upstream side of the aerosol-generating article may be inserted inside the body 20, and a downstream side of the aerosol-generating article may be exposed to the outside of the body 20. The user may inhale the aerosol with the externally exposed portion of the aerosol-generating article in the mouth.

The power source 21 may supply power to operate the components of the aerosol generation device 2. The power source 21 may be a battery. The power source 21 may supply power to at least one of the controller 22 or the surface wave generation part 28.

The controller 22 may control the overall operation of the aerosol generation device 2. The controller 22 may be mounted on a PCB. The controller 22 may control the operation of at least one of the power source 21or the surface wave generation part 28. The controller 22 may also control the operation of a display, a motor, or the like installed in the aerosol generation device 2. The controller 22 may determine a state of each of the components of the aerosol generation device 2 to determine whether the aerosol generation device 2 is in an operable state.

In one embodiment, the storage part 29 may store at least one aerosol-forming substrate. For example, the aerosol-forming substrate may include an aerosol-generating material in any one of various states including, for example, a liquid state, a solid state, a gaseous state, a gel state, or the like. The aerosol-generating material may include a liquid composition. For example, the liquid composition may be a liquid containing a tobacco-containing substance that contains a volatile tobacco flavor component, or a liquid containing a non-tobacco substance.

As the storage part 29 and/or the surface wave generation part 28 operates by an electrical signal or a wireless (or radio) signal or the like transmitted from the controller 22, a phase of the aerosol-forming substrate in the storage part 29 may be changed into a phase of gas by the surface wave generation part 28, generating an aerosol. The aerosol may refer to gas in a state where vaporized particles generated from the aerosol-forming substrate and air are mixed.

The storage part 29 may store a functional material which may constitute the aerosol-forming substrate. The functional material may be stored inside the storage part 29 in at least one of the following forms: a gas phase, a liquid phase, and a solid phase. The functional material may include, for example, nicotine, glycerin, propylene glycol, flavorings such as menthol, or medications for treating respiratory diseases such as asthma, chronic obstructive pulmonary disease (COPD), or the like, oils such as aromas, caffeine, taurine, vaccines, or the like. The functional material is, however, not limited to the examples listed above, but may include various substances. In one embodiment, the storage part 29 may be provided as a plurality of storage parts, and the same functional material or different functional materials may be stored in the plurality of storage parts 29. For example, in a case where the same functional material is stored in the plurality of storage parts 29, and one of the plurality of storage parts 29 is fully used, remaining storage parts 29 may serve as a spare. For example, in a case where the different functional materials are stored in the plurality of storage parts 29, any one of the plurality of storage parts 29 may be selected for aerosolization according to the preference of the user.

The storage part 29 may be disposed adjacent to an atomization area (e.g., an atomization area AA of FIG. 7) within the body 20. The storage part 29 may be replaceable and, for example, the storage part 29 may be removably coupled to the side surface 203. Accordingly, the storage part 29 may be replaceable from the side surface of the body 20 that forms the outer shape of the aerosol generation device 2, providing the user with convenience.

In one embodiment, between the storage part 29 and the atomization area, a microchannel forming part (not shown) that provides the aerosol-forming substrate from the storage part 29 to the atomization area may be further included. The microchannel forming part may include a microfluidic inlet connected to one side of the storage part 29 and a microfluidic outlet facing the atomization area.

The microchannel forming part may actively and quantitatively supply a highly viscous liquid (e.g., the aerosol-forming substrate) to the atomization area. For example, the microchannel forming part may be formed with a microchannel that is formed of polydimethylsiloxane (PDMS), which is a transparent elastomer, by photolithography and soft lithography processes. The microfluidic inlet of the microchannel forming part may be connected to the storage part 29 via a tube, and may quantitatively supply a desired flow rate of the liquid (e.g., the aerosol-generating substrate) by a pump. Between the pump and the microfluidic inlet, a microfluidic connector may be provided, and the microfluidic connector may prevent the tube from being an obstacle to the movement of the liquid. The width and height of the microfluidic outlet may be set to maintain the thickness of a liquid film of the liquid (e.g., the aerosol-forming substrate) to be small. The main operating variables in surface wave-induced aerosolization may include physical properties (e.g., viscosity, density, surface tension, etc.) of the liquid, the width and height of the liquid film, and the frequency and intensity of an applied surface wave, and based on these, the width and height of the microfluidic outlet may be set. For example, the width and height of the microfluidic outlet may be set to 200 micrometers (µm) and 100 µm.

The surface wave generation part 28 may be connected to the atomization area and may generate surface waves. The surface wave generation part 28 will be described in detail below with reference to FIGS. 7 through 9.

Referring to FIG. 7, the surface wave generation part 28 may include a piezoelectric plate 281 connected to the atomization area AA, and a transducer 282 disposed on the piezoelectric plate 281.

The piezoelectric plate 281 may include a piezoelectric material that interconverts electrical energy and mechanical energy. The piezoelectric plate 281 may form a surface over which the surface waves are transmitted.

The transducer 282 (e.g., an interdigital transducer) may be disposed on the piezoelectric plate 281 and, for example, may be deposited on the piezoelectric plate 281. The transducer 282 may convert an electrical signal transmitted from the power source 21 and/or the controller 22 into a surface wave. For example, the transducer 282 may be patterned on the surface of the piezoelectric material in an interdigitated finger (or comb-patterned) form by a semiconductor etching process. In this example, when an AC voltage having an operating frequency corresponding to a spacing of the transducer 282 is applied to the transducer 282, mechanical contraction and expansion of the piezoelectric material of the piezoelectric plate 281 may occur, generating a surface wave that travels along the surface of the piezoelectric plate 281. The surface wave generated via the transducer 282 may form an acoustic field within the atomization area AA to aerosolize the aerosol-forming substrate from a storage part (e.g., the storage part 29 of FIG. 6) provided on the atomization area AA. An aerosol generated by the surface wave may be fine and uniform in particle size.

In one embodiment, the transducer 282 may be provided as a plurality of transducers. In this case, for example, the transducer 282 may include a first transducer 282a and a second transducer 282b. The first transducer 282a and the second transducer 282b may be disposed opposite each other to face each other, with the atomization area AA therebetween. Traveling surface waves generated by the respective two transducers (e.g., the first transducer 282a and the second transducer 282b) may meet to form a standing wave, such that the amplitude of the phase-unified surface wave may be maximized. For example, a spacing between the two transducers (e.g., the first transducer 282a and the second transducer 282b) may be designed to be approximately 65 to 200λ, such that standing surface wave may be well formed.

In one embodiment, the surface wave generation part 28 may further include the reflector 283 configured to reflect the surface wave from the transducer 282. The reflector 283 may be disposed on an opposite side of the atomization area AA with the transducer 282 therebetween. The reflector 283 may concentrate the traveling surface wave generated in two directions (e.g., +/- Y-directions in FIG. 7) by the transducer 282 into one direction (e.g., the -Y-direction relative to the first transducer 282a and the +Y-direction relative to the second transducer 282b, in FIG. 7). The reflector 283 may maximize the amplitude of the surface wave being used. The reflector 283 may be configured as a close type that is most efficient in reflecting waves.

Referring to FIG. 8, the transducer 282 (e.g., the first transducer 282a) and the reflector 283 may be spaced apart, and a spacing distance d therebetween may be set to correspond to a width of an electrode finger (e.g., a first electrode finger 28211 or a second electrode finger 28221 of FIG. 9) of the transducer 282. For example, the spacing distance d, which is a distance between an electrode end of the transducer 282 and the reflector 283, may be designed to be a minimum value such that it is one quarter (1/4) of a wavelength, minimizing attenuation of a sound wave on a substrate (e.g., the piezoelectric plate 281 of FIG. 7).

Referring to FIG. 9, the transducer 282 may include a first electrode set 2821 and a second electrode set 2822. As the transducer 282 includes the first electrode set 2821 and the second electrode set 2822, the transducer 282 may have a comb-patterned shape, such as, an interdigitated finger shape. For example, the transducer 282 may be designed to include electrodes in the comb-patterned shape in which an electrode width λ/4 and an electrode spacing λ/4 are the same, to maximize the energy efficiency of a surface wave generation element.

The first electrode set 2821 may include a plurality of first electrode fingers 28211 extending toward a second bus bar 28222 of the second electrode set 2822. The second electrode set 2822 may include a plurality of second electrode fingers 28221 extending toward a first bus bar 28212 of the first electrode set 2821. The first electrode fingers 28211 and the second electrode fingers 28221 may be arranged alternately.

In one embodiment, the first electrode fingers 28211 and the second electrode fingers 28221 may be spaced at equidistance intervals (λ/4)*.* At least one of a width (λ/4) of a first electrode finger 28211 or a width *(*λ/4) of a second electrode finger 28221 may be equal to a spacing distance (λ/4) between the first electrode finger 28211 and the second electrode finger 28221.

The first electrode set 2821 may further include the first bus bar 28212 to which the first electrode fingers 28211 are connected and a first electrode pad 28213 connected to one end of the first bus bar 28212. The second electrode set 2822 may further include the second bus bar 28222 to which the second electrode fingers 28221 are connected and a second electrode pad 28223 connected to one end of the second bus bar 28222. The first bus bar 28212 and the second bus bar 28222 may be parallel to each other. For example, the first bus bar 28212 may extend from the first electrode pad 28213 toward an atomization area (e.g., the atomization area AA of FIG. 7), and the second bus bar 28222 may extend from the second electrode pad 28223 toward the atomization area (e.g., the atomization area AA of FIG. 7).

In one embodiment, the transducer 232 may be fabricated using a five-layer thin metal film of titanium (Ti)/aluminum (Al)/silicon dioxide (SiO2)/titanium (Ti)/aluminum (Al) to maximize the energy efficiency of the surface wave generated. In this case, the silicon dioxide layer may serve as an insulating layer to protect the electrodes. The thickness of a pad portion (e.g., the first pad 28213 and/or second pad 28223) and/or a bus bar portion (e.g., the first bus bar 28212 and/or second bus bar 28222) may be deposited to be thicker than the thickness of an electrode portion (e.g., the first electrode fingers 28211 and/or second electrode fingers 28221) of the transducer 282 to ensure that an applied electric field is as well formed as possible only on the transducer 282.

For example, the transducer 282 may be fabricated using processes such as the following ones based on a photomask. A cleaning process for an LN (LiNbO₃) wafer may be performed using the most durable black LN wafer to avoid substrate damage due to a high voltage involved in implementing liquid phase atomization and using a solution of sulfuric acid and hydrogen peroxide. Subsequently, a photolithography process may be performed on the cleaned wafer using a negative photoresist (Negative PR), and in this case, using the primary metal patterning photomask presented above, the photoresist may be remained in portions excluding a transducer area. In addition, in a develop process, the photoresist in the transducer area may be removed. Subsequently, primary metal application may be performed by electronic-beam (E-beam) evaporation, and then a lift-off process may be performed to remove the metal and the photoresist from an area excluding the transducer area. Subsequently, a process of forming an insulating layer of SiO2 may be performed using the silicon dioxide patterning mask to prevent damage to a transducer from an atomized liquid. Subsequently, secondary metal application may be performed using a secondary metal patterning photomask to minimize the resistance formed in the transducer after the insulating layer is formed. In this case, the overall process may be the same as the primary metal application, but the secondary metal application may be performed such that the pad portion of the transducer has a higher height.

For a heating-type aerosol generation device (e.g., a heated inhaler), a temperature condition that requires a temperature of approximately 250 degrees (°) may be required, and a deterioration of a liquid inhalant used, which is triggered by a high temperature, may be a challenging concern. The aerosol generation device 2 of one or more embodiments described herein may atomize a highly viscous liquid within a temperature of approximately 100° and may thereby prevent the aerosol-forming substrate in the liquid phase from deteriorating.

According to embodiments, the aerosol generation device 2 may reduce noise generation, achieve abundant atomization, and generate uniform and small atomization particles.

According to one embodiment, an aerosol generation device 2 may include: a body 20 including a first surface 201, a second surface 202 opposite the first surface 201, and a side surface 203 between the first surface 201 and the second surface 202, and including a mouthpiece end (ME) disposed on the first surface 201; an atomization area AA disposed within the body 20 and connected to the mouthpiece end ME; a storage part 29 connected to the atomization area AA and storing an aerosol-forming substrate; and a surface wave generation part 28 connected to the atomization area AA and generating surface waves. The surface wave generation part 28 may include: a piezoelectric plate 281 extending into the atomization area AA and a transducer 282 disposed on the piezoelectric plate 281. The transducer 282 may convert an electrical signal into a surface wave that is to be provided to the atomization area AA, and the transducer 282 may have a comb-patterned shape.

**In** one embodiment, the transducer 282 may include a first electrode set 2821 and a second electrode set 2822 facing the first electrode set 2821. The first electrode set 2821 may include a plurality of first electrode fingers 28211 extending toward the second electrode set 2822, and the second electrode set 2822 may include a plurality of second electrode fingers 28221 extending toward the first electrode set 2821. The first electrode fingers 28211 and the second electrode fingers 28221 may be arranged alternately.

The first electrode set 2821 may further include a first bus bar 28212 to which the first electrode fingers 28211 are connected and a first electrode pad 28213 connected to one end of the first bus bar 28212, and the second electrode set 2822 may further include a second bus bar 28222 to which the second electrode fingers 28221 are connected and a second electrode pad 28223 connected to one end of the second bus bar 28222. The first bus bar 28212 and the second bus bar 28222 may be parallel to each other.

The first bus bar 28212 may extend from the first electrode pad 28213 toward the atomization area AA, and the second bus bar 28222 may extend from the second electrode pad 28223 toward the atomization area AA.

**In** one embodiment, the first electrode fingers 28211 and the second electrode fingers 28221 may be equidistantly spaced apart.

At least one of a width of a first electrode finger 28211 or a width of a second electrode finger 28221 may be equal to a spacing distance between the first electrode finger 28211 and the second electrode finger 28221.

The surface wave generation part 28 may further include a reflector 283 configured to reflect the surface wave from the transducer 282, and the reflector 283 may be disposed on an opposite side of the atomization area AA with the transducer 282 therebetween.

The transducer 282 may be provided in a pair of transducers, and the transducers 282 of the pair may be disposed with the atomization area AA therebetween.

**In** one embodiment, between the storage part 29 and the atomization area AA, a microchannel forming part configured to provide the aerosol-forming substrate from the storage part 29 to the atomization area AA may be further included. The microchannel forming part may include a microfluidic inlet connected to one side of the storage part 29 and a microfluidic outlet facing the atomization area AA.

According to one embodiment, an aerosol generation device 2 may include: a body 20 including a first surface 201, a second surface 202 opposite the first surface 201, and a side surface 203 between the first surface 201 and the second surface 202, and including a mouthpiece end (ME) disposed on the first surface 201; an atomization area AA disposed within the body 20 and connected to the mouthpiece end ME; a storage part 29 connected to the atomization area AA and storing an aerosol-forming substrate; and a surface wave generation part 28 connected to the atomization area AA and generating surface waves. The surface wave generation part 28 may include: a piezoelectric plate 281 extending into the atomization area AA; a transducer 282 disposed on the piezoelectric plate 281; and a reflector 283 disposed on an opposite side of the atomization area AA with the transducer 282 therebetween. The transducer 282 may convert an electrical signal into a surface wave that is to be provided to the atomization area AA, and the reflector 283 may reflect the surface wave from the transducer 282 to the atomization area AA.

In one embodiment, the transducer 282 may be provided as a pair of transducers, and the transducers 282 of the pair may be disposed with the atomization area AA therebetween.

The transducer 282 and the reflector 283 may be spaced apart from each other.

A spacing distance between the transducer 282 and the reflector 283 may be set to correspond to a width of an electrode finger 28211 or 28221 of the transducer 282.

The descriptions of the above-described embodiments are provided merely as examples, and it will be understood by a person of ordinary skill in the art that various changes and equivalents may be made thereto. Therefore, the scope of the disclosure should be defined by the appended claims, and all differences within the scope equivalent to those described in the claims will be construed as being included in the scope of protection defined by the claims.

## Claims

1. An aerosol generation device, comprising:
a body comprising a first surface, a second surface opposite the first surface, and a side surface between the first surface and the second surface, and comprising a mouthpiece end disposed on the first surface;
an atomization area disposed within the body and connected to the mouthpiece end;
a storage part connected to the atomization area and storing therein an aerosol-forming substrate; and
a surface wave generation part connected to the atomization area and generating surface waves,
wherein the surface wave generation part comprises:
a piezoelectric plate extending into the atomization area; and
a transducer disposed on the piezoelectric plate,
wherein the transducer is configured to convert an electrical signal into a surface wave, wherein the surface wave is provided to the atomization area and the transducer has a comb pattern shape.

2. The aerosol generation device of claim 1, wherein the transducer comprises:
a first electrode set; and
a second electrode set facing the first electrode set,
wherein the first electrode set comprises a plurality of first electrode fingers extending toward the second electrode set, and
the second electrode set comprises a plurality of second electrode fingers extending toward the first electrode set,
wherein the first electrode fingers and the second electrode fingers are arranged alternately.

3. The aerosol generation device of claim 2, wherein the first electrode set further comprises:
a first bus bar to which the first electrode fingers are connected, and a first electrode pad connected to one end of the first bus bar, and
the second electrode set further comprises:
a second bus bar to which the second electrode fingers are connected, and a second electrode pad connected to one end of the second bus bar,
wherein the first bus bar and the second bus bar are parallel to each other.

4. The aerosol generation device of claim 3, wherein the first bus bar extends from the first electrode pad toward the atomization area, and
the second bus bar extends from the second electrode pad toward the atomization area.

5. The aerosol generation device of claim 2, wherein the first electrode fingers and the second electrode fingers are equidistantly spaced apart.

6. The aerosol generation device of claim 5, wherein at least one of a width of a first electrode finger or a width of a second electrode finger is equal to a spacing distance between the first electrode finger and the second electrode finger.

7. The aerosol generation device of claim 1, wherein the surface wave generation part further comprises:
a reflector configured to reflect the surface wave from the transducer,
wherein the reflector is disposed on an opposite side of the atomization area with the transducer therebetween.

8. The aerosol generation device of claim 7, wherein the transducer is provided as a pair of transducers, wherein the transducers of the pair are disposed with the atomization area therebetween.

9. The aerosol generation device of claim 1, further comprising:
a microchannel forming part, between the storage part and the atomization area, configured to provide the aerosol-forming substrate from the storage part to the atomization area,
wherein the microchannel forming part comprises:
a microfluidic inlet connected to one side of the storage part; and
a microfluidic outlet facing the atomization area.

10. An aerosol generation device, comprising:
a body comprising a first surface, a second surface opposite the first surface, and a side surface between the first surface and the second surface, and comprising a mouthpiece end disposed on the first surface;
an atomization area disposed within the body and connected to the mouthpiece end;
a storage part connected to the atomization area and storing therein an aerosol-forming substrate; and
a surface wave generation part connected to the atomization area and generating surface waves,
wherein the surface wave generation part comprises:
a piezoelectric plate extending into the atomization area;
a transducer disposed on the piezoelectric plate; and
a reflector disposed on an opposite side of the atomization area with the transducer therebetween,
wherein the transducer is configured to convert an electrical signal into a surface wave, wherein the surface wave is provided to the atomization area and the reflector is configured to reflect the surface wave from the transducer into the atomization area.

11. The aerosol generation device of claim 10, wherein the transducer is provided as a pair of transducers,
wherein the transducers of the pair are disposed with the atomization area therebetween.

12. The aerosol generation device of claim 10, wherein the transducer and the reflector are spaced apart from each other.

13. The aerosol generation device of claim 12, wherein a spacing distance between the transducer and the reflector is set to correspond to a width of an electrode finger of the transducer.
